# EUROPEAN PATENT APPLICATION

(11) **EP 3 120 772 A1**
(43) Date of publication of application: **25.01.2017**
(21) Application number: 16181116.1
(22) Date of filing: 25.07.2016
(51) Int. Cl.: A61B 6/00

(54) **IMAGING CONSOLE AND RADIATION IMAGING SYSTEM**

(30) Priority: 24.07.2015 JP 2015146695
(71) Applicant: Konica Minolta, Inc., Tokyo 100-7015 (JP)
(72) Inventor: Tezuka, Hidetake, Tokyo 100-7015 (JP); Haraguchi, Tsuyoshi, Tokyo 100-7015 (JP); Hosoki, Tetsu, Tokyo 100-7015 (JP)
(74) Representative: Henkel, Breuer & Partner

(57) **Abstract**

An imaging console (2) including: a display section (24) which is capable of displaying a moving image; and a control section (21) which performs control on the basis of imaging order information regarding imaging to be performed, the imaging order information specifying at least patient information (P2 to P6) regarding a patient and imaging site information (P7 and P8) regarding an imaging site; wherein in a case where the imaging order information specifies imaging of a moving image, at the time when the imaging order information is obtained or input, the control section (21) obtains a past moving image of the patient or a past moving image of the imaging site on the basis of at least one of the patient information (P2 to P6) and the imaging site information (P7 and P8), and displays the obtained past moving image on the display section (24).

## Description

### BACKGROUND OF THE INVENTION

### 1. Field of the Invention

The present invention relates to an imaging console and a radiation imaging system.

### 2. Description of Related Art

Semiconductor image sensors such as FPDs (flat panel detectors) have been developed as radiation apparatuses for capturing still images instead of conventional films/screens, stimulable phosphor plates and such like. There have been attempts to use such semiconductor image sensors for capturing dynamic images (images of dynamic states) at imaging sites (examination target sites) to use the dynamic images for diagnosis (for example, see Japanese Patent Application Laid Open Publication No. 2011-152154 and International Publication NO. WO 2013/058055).

Specifically, by using the rapid responsiveness of semiconductor image sensors of reading out image data and resetting residual electric charge, pulsed radiation is continuously emitted from a radiation source at the reading/deleting timings of semiconductor image sensors, and imaging is performed normally a plurality of times per second to capture an image of a dynamic state at an imaging site. By sequentially displaying a series of images captured by the imaging and images having superposed analysis results of the captured images, for example, a doctor can recognize a series of moves at the imaging site.

Several techniques are proposed to appropriately perform dynamic imaging. For example, Japanese Patent Application Laid Open Publication No. 2011-152154 describes a system in which pre-imaging is performed before actual imaging, a plurality of dynamic images captured at the pre-imaging is analyzed to calculate an imaging condition for actual imaging, and the actual imaging is performed on the basis of the calculated imaging condition. International Publication NO. WO 2013/058055 describes a system which facilitates appropriate imaging by notifying a user of an appropriate timing of imaging in the continuous breathing motion for the present imaging on the basis of past imaging.

When the above-described semiconductor image sensors are used in a field such as simple imaging (also called general imaging or such like, that is, imaging of capturing a single image by emitting radiation once from a radiation source), since the simple imaging has a long history and has established desired images as images for diagnosis, the imaging can be performed appropriately by extracting an imaging condition of imaging in past imaging which was performed appropriately, and applying the imaging condition to the present imaging as described in Japanese Patent Application Laid Open Publication No. 2013-048746, for example.

On the other hand, the above techniques for analyzing dynamic states, that is, the techniques attempting to use semiconductor image sensors to capture dynamic images at imaging sites in subjects and use the images for diagnosis are new examination methods which have a short history. Thus, references indicating desired images for diagnosis are not presently established (or do not exist).

### SUMMARY OF THE INVENTION

The present invention has been made in consideration of the above problems, and an object of the present invention is to provide an imaging console and a radiation imaging system which can perform imaging appropriately by using a past moving image and such like as a reference.

In order to solve the above problems, according to one aspect of a preferred embodiment of the present invention, there is provided an imaging console and a radiation imaging system which includes the imaging console, the imaging console including: a display section which is capable of displaying a moving image; and a control section which performs control on the basis of imaging order information regarding imaging to be performed, the imaging order information specifying at least patient information regarding a patient and imaging site information regarding an imaging site; wherein in a case where the imaging order information specifies imaging of a moving image, at the time when the imaging order information is obtained or input, the control section obtains a past moving image of the patient or a past moving image of the imaging site on the basis of at least one of the patient information and the imaging site information specified in the imaging order information, and displays the obtained past moving image on the display section.

### BRIEF DESCRIPTION OF THE DRAWINGS

The above and other objects, advantages and features of the present invention will become more fully understood from the detailed description given hereinafter and the appended drawings which are given by way of illustration only, and thus are not intended as a definition of the limits of the present invention, and wherein:
FIG. 1 is a view showing a configuration of medical information management system;
FIG. 2 is a view showing a configuration of an example of a radiation imaging system in an embodiment;
FIG. 3 is a flowchart showing imaging control processing executed by a control section of an imaging console;
FIG. 4 is a flowchart showing image analysis processing executed by a control section of a diagnostic console;
FIG. 5 is a view showing frame images of a plurality of time phases captured in one respiratory cycle;
FIG. 6 is a graph showing temporal change of height in vertical direction of diaphragm;
FIG. 7 is a view showing a configuration example of imaging console; and
FIG. 8 is a view showing an example of imaging order information.

### DETAILED DESCRIPTION OF THE PREFERRED EMBODIMENT

Hereinafter, an embodiment of an imaging console and a radiation imaging system according to the present invention will be described in detail with reference to the drawings. However, the present invention is not limited to the illustrated examples.

According to the type of console and the radiation imaging system in the embodiment, imaging can be performed appropriately by using a past moving image and such like as a reference.

Hereinafter, the present invention is explained for a case where moving image capturing is dynamic imaging. However, the present invention is not limited to this, and can also be applied to other cases of moving image capturing in which a plurality of images (frame images) is temporally continuously captured by tomosynthesis and such like, for example. The moving image capturing in this case is a kind of restricted moving image capturing and different from normal moving image capturing in respects of time limitation and assurance of real time display.

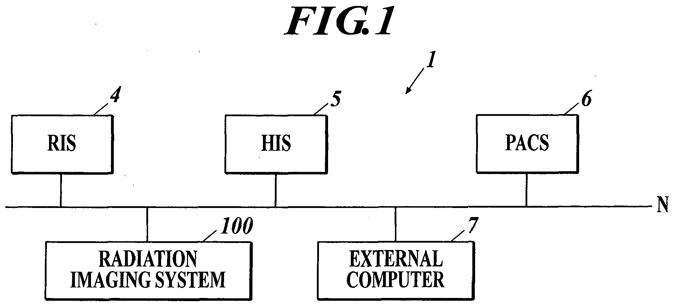

As shown in FIG. 1, the radiation imaging system 100 in the embodiment is connected to a medical information management system 1 via a network N, the medical information management system 1 including an external system such as an RIS (Radiology Information System) 4, an HIS (Hospital Information System) 5 and PACS (Picture Archiving and Communication System) 6, and an external computer 7 such as a server. Alternatively, the radiation imaging system 100 is configured as a part of the medical information management system 1.

### [Configuration of Radiation imaging system]

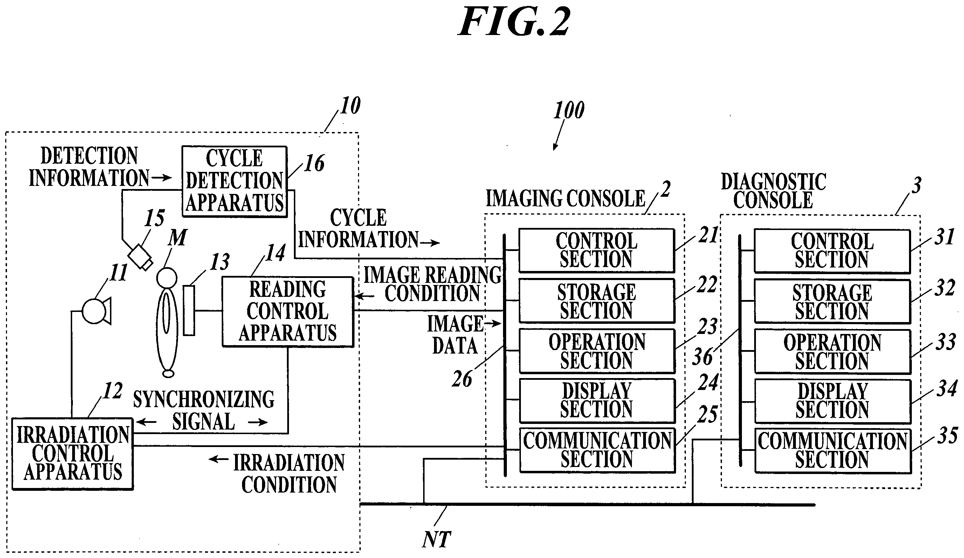

Hereinafter, an example of the radiation imaging system 100 in the embodiment will be described by taking, as an example, a case of capturing dynamic images. FIG. 2 shows a configuration example of radiation imaging system 100 in this case. As shown in FIG. 2, the radiation imaging system 100 includes an imaging console 2 used by an imaging operator for imaging and a diagnostic console 3 used by a doctor for diagnosis. An imaging apparatus 10 is connected to the imaging console 2 via a communication cable or the like, and the imaging console 2 is connected to the diagnostic console 3 via a communication network NT such as a LAN (Local Area Network).

### [Configuration of Imaging apparatus 10]

The imaging apparatus 10 captures images of dynamic states of chest having a cycle such as the change of lung state of expansion and contraction according to a breathing motion and the heartbeat, for example. Dynamic imaging is performed by continuously emitting radiation such as X-ray to a chest of a human body and obtaining a plurality of images (that is, continuous imaging). A series of images obtained by the continuous imaging is referred to as a dynamic image. The images forming the dynamic image are referred to as frame images.

Not only the dynamic states of lung and heart as described above, the radiation imaging system 100 in the embodiment may be configured to also capture images of dynamic states regarding bending and stretching of joints in a human body to analyze bending and stretching function of the joints, for example.

As shown in FIG. 2, the imaging apparatus 10 is configured by including a radiation source 11, an irradiation control apparatus 12, a radiation detection section 13, a reading control apparatus 14, a cycle detection sensor 15, a cycle detection apparatus 16 and such like. The radiation detection section 13 and the reading control apparatus 14 may be integrally formed (that is, the radiation detection section 13 may be configured to include the reading control apparatus 14).

The radiation source 11 emits radiation (X-ray) to a subject M in accordance with control of the irradiation control apparatus 12. The irradiation control apparatus 12 is connected to the imaging console 2, and controls the radiation source 11 to perform radiation imaging on the basis of an irradiation condition input from the imaging console 2. The irradiation condition input from the imaging console 2 is, for example, a pulse rate, a pulse width, a pulse interval, imaging start/end timing, a value of X-ray tube current, a value of X-ray tube voltage and a type of filter when irradiation is continuously performed. The pulse rate is the number of irradiation per second and consistent with an after-mentioned frame rate. The pulse width is an irradiation time required for one irradiation. The pulse interval is a time from start of one irradiation to start of next irradiation in the continuous imaging, and consistent with an after-mentioned frame interval.

The radiation detection section 13 is configured by including a semiconductor image sensor such as an FPD. The FPD has a glass substrate, for example, and a plurality of pixels is arranged in matrix at a predetermined position on the substrate for detecting at least radiation which was emitted from the radiation source 11 and has transmitted through the subject M according to the intensity and converting the detected radiation into electric signals to be accumulated. Each pixel is formed of a switching section such as a TFT (Thin Film Transistor), for example.

The reading control apparatus 14 is connected to the imaging console 2. The reading control apparatus 14 controls the switching sections of respective pixels in the radiation detection section 13 on the basis of an image reading condition input from the imaging console 2, switches the reading of electric signals accumulated in the pixels, and reads out the electric signals accumulated in the radiation detection section 13 to obtain image data. The image data is a frame image. The reading control apparatus 14 outputs the obtained frame image to the imaging console 2.

The image reading condition includes a frame rate (or frame interval), a pixel size, an image size (matrix size) and such like. The frame rate is the number of frame images obtained per second and consistent with the pulse rate. The frame interval is a time from start of obtaining one frame image to start of obtaining the next frame image, and consistent with the pulse interval.

Here, the irradiation control apparatus 12 and the reading control apparatus 14 are connected to each other, and transmit synchronizing signals to each other to synchronize the irradiation operation with the image reading operation.

The cycle detection sensor 15 detects the state of breathing motion of subject M, and outputs the detection information to the cycle detection apparatus 16. As the cycle detection sensor 15, a breathing monitor belt, a CCD (Charge Coupled Device) camera, an optical camera and a spirometer can be applied, for example.

The cycle detection apparatus 16 detects the number of respiratory cycles and the present state in one cycle of breathing motion (the state is one of inspiration, turning point from inspiration to expiration, expiration and turning point from expiration to inspiration, for example) on the basis of the detection information input by the cycle detection sensor 15. The cycle detection apparatus 16 outputs the detection result (cycle information) to the control section 21 of the imaging console 2. The cycle detection apparatus 16 sets a start point of one cycle to be, for example, the timing when the cycle detection sensor 15 (such as breathing monitor belt, CCD camera, optical camera and spirometer) inputs detection information indicating the turning point from the inspiration to expiration of lung state. The cycle detection apparatus 16 recognizes one cycle to be a period from the start point to a timing when this state is detected next.

### [Configuration of Imaging console 2]

Next, a general configuration of the imaging console 2 will be described. The detailed configuration of imaging console 2 in the embodiment will be described later.

The imaging console 2 outputs the irradiation condition and the image reading condition to the imaging apparatus 10, controls the radiation imaging and reading operation of radiation images by the imaging apparatus 10, and displays the dynamic image obtained by the imaging apparatus 10 for an imaging operator to confirm positioning and whether the image is appropriate for diagnosis.

As shown in FIG. 2, the imaging console 2 is configured by including a control section 21, a storage section 22, an operation section 23, a display section 24 and a communication section 25, which are connected to each other via a bus 26.

The control section 21 is configured by including a CPU (Central Processing Unit), a RAM (Random Access Memory) and such like. According to the operation of operation section 23, the CPU of the control section 21 reads out system programs and various processing programs stored in the storage section 22 to load the programs into the RAM, executes various types of processing including after-mentioned imaging control processing in accordance with the loaded program, and integrally controls the operations of sections in the imaging console 2 and the irradiation operation and reading operation of the imaging apparatus 10.

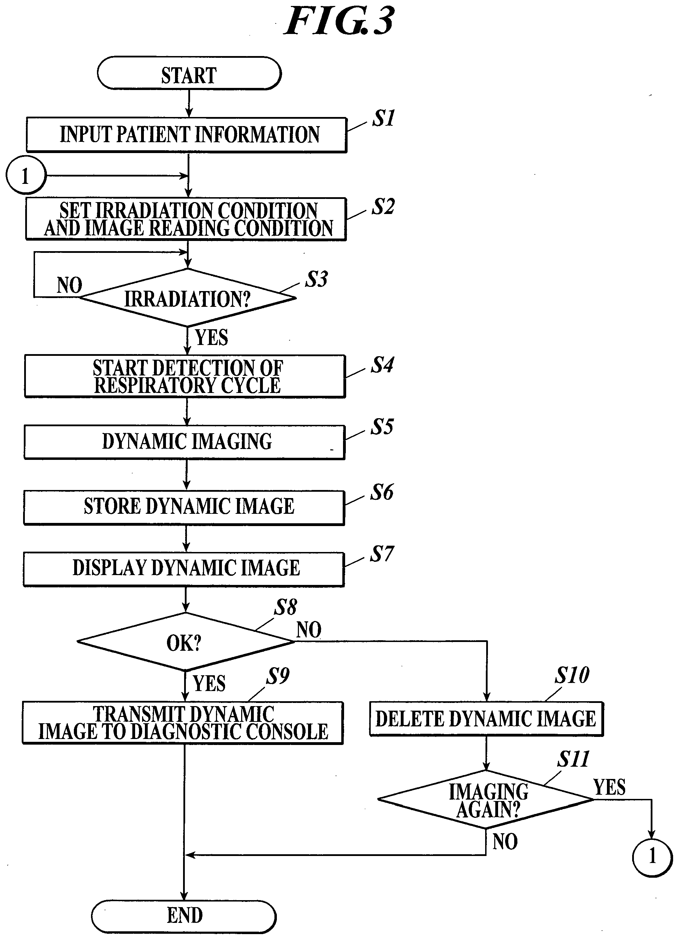

The storage section 22 is configured by including a non-volatile semiconductor memory and a hard disk. The storage section 22 stores various programs executed by the control section 21, parameters necessary for executing processing by the programs, and data of processing results. For example, the storage section 22 stores an imaging control program for executing the imaging control processing shown in FIG. 3. The storage section 22 stores the irradiation condition and the image reading condition so as to be associated with the examination target site. The various programs are stored in a form of readable program code, and the control section 21 executes the operation according to the program code as needed.

The operation section 23 is configured by including a keyboard including cursor keys, numeric keys and various function keys and a pointing device such as a mouse. The operation section 23 outputs an instruction signal input by a key operation to the keyboard or a mouse operation to the control section 21. The operation section 23 may include a touch panel on the display screen of display section 24. In this case, the operation section 23 outputs the input instruction signal to the control section 21 via the touch panel.

The display section 24 is configured by a monitor such as an LCD (Liquid Crystal Display) and a CRT (Cathode Ray Tube), and replays and displays the dynamic image and displays various types of data and instructions input from the operation section 23 in accordance with an instruction of a display signal input from the control section 21.

The communication section 25 includes a LAN adapter, a modem, a TA (Terminal Adapter) and such like, and controls the data transmission and reception with the apparatuses connected via the communication network NT.

### [Configuration of Diagnostic console 3]

The diagnostic console 3 is a moving image processing apparatus for obtaining the dynamic image from the imaging console 2 and displaying the obtained dynamic image for a doctor to perform diagnosis by interpretation of radiogram. In some cases, the diagnostic console 3 obtains the dynamic image from an external system after the dynamic image is once transmitted from the imaging console 2 to the external system such as PACS (Picture Archiving and Communication System). The imaging console 2 and the diagnostic console 3 may be integrally configured, that is, a single apparatus may function as both the imaging console 2 and the diagnostic console 3.

As shown in FIG. 2, the diagnostic console 3 is configured by including a control section 31, a storage section 32, an operation section 33, a display section 34 and a communication section 35, which are connected to each other via a bus 36.

The control section 31 is configured by including a CPU and a RAM. According to the operation of the operation section 33, the CPU of the control section 31 reads out system programs and various processing programs stored in the storage section 32 to load them into the RAM, executes the various types of processing including after-mentioned image analysis processing in accordance with the loaded program, and integrally controls the sections of the diagnostic console 3. The control section 31 achieves an image analysis section by executing the after-mentioned image analysis processing.

The storage section 32 is configured by including a non-volatile semiconductor memory and a hard disk. The storage section 32 stores various programs including an image analysis program for executing the image analysis processing by the CPU 31, parameters necessary for executing processing by the programs and data of processing results. The various programs are stored in a form of readable program code, and the control section 31 executes the operation according to the program code as needed.

The operation section 33 is configured by including a keyboard including cursor keys, numeric keys and various function keys and a pointing device such as a mouse, and outputs an instruction signal input by a key operation to the keyboard and a mouse operation to the control section 31. The operation section 33 may include a touch panel on the display screen of the display section 34. In this case, the operation section 33 outputs an instruction signal, which was input via the touch panel, to the control section 31.

The display section 34 is configured by including a monitor such as an LCD and a CRT, replays and displays dynamic images and displays various data and instructions input from the operation section 33 in accordance with the instruction of display signal input from the control section 31.

The communication section 35 includes a LAN adapter, a modem, a TA and such like, and controls data transmission and reception with the apparatuses connected to the communication network NT.

### [Operation of Radiation imaging system 100]

Next, the operation of the radiation imaging system 100 will be described.

### [Operations of Imaging apparatus 10 and Imaging console 2]

First, imaging operation by the imaging apparatus 10 and general imaging operation by the imaging console 2 when imaging is performed will be described. FIG. 3 shows imaging control processing executed by the control section 21 in the imaging console 2. The imaging control processing is executed in cooperation between the control section 21 and the imaging control program stored in the storage section 22.

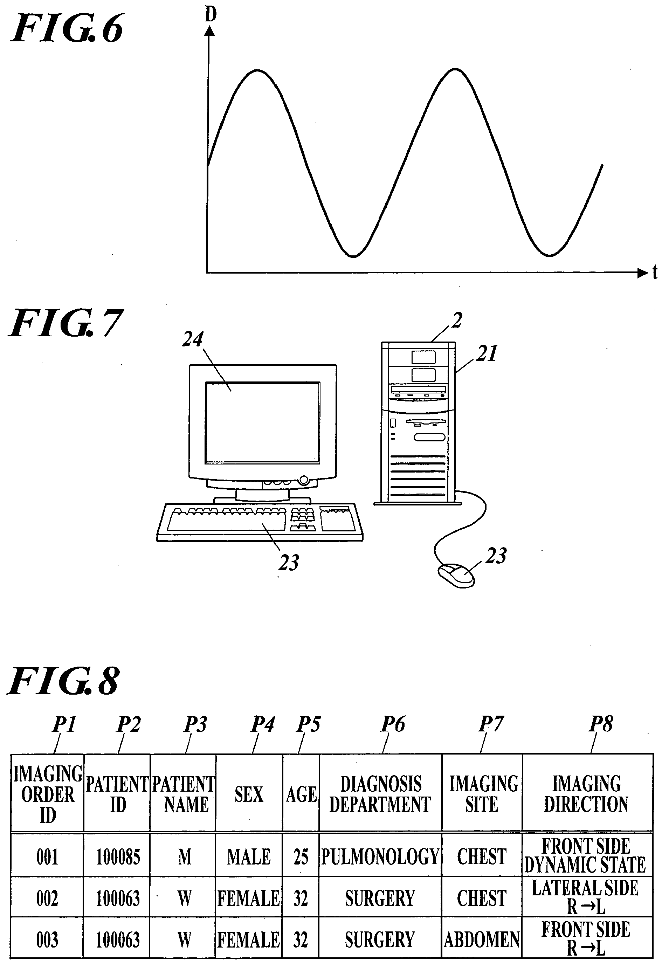

Patient information (patient name, age, sex and such like) of the imaging target (subject M) is input to the imaging console 2 by the operation section 23 of the imaging console 2 obtaining imaging order information (see FIG. 8 to be described later) from the RIS 4 (see FIG. 1) (step S1). Next, the irradiation condition is read out from the storage section 22 and set in the irradiation control apparatus 12, and the image reading condition is read out from the storage section 22 and set in the reading control apparatus 14 (step S2).

The following is an example of dynamic imaging. That is, when the imaging operator operates an exposure switch not shown in the drawings of the irradiation control apparatus 12 (see FIG. 2) to input a irradiation instruction (step S3; YES), the instruction to start cycle detection is output to the cycle detection apparatus 16, and the cycle detection of breathing motion of the subject M is started by the cycle detection sensor 15 and the cycle detection apparatus 16 (step S4). Then, the dynamic imaging is performed (step S5), and the imaging operation is stopped when a predetermined number of dynamic state cycles is detected by the cycle detection apparatus 16, for example.

The frame images obtained by the imaging are sequentially input to the imaging console 2, and stored in the storage section 22 so as to be associated with the respective numbers indicating the order of imaging (step S6). The frame images are displayed on the display section 24 (step S7). The imaging operator confirms the positioning and such like by the displayed dynamic image, and determines whether an image appropriate for diagnosis was obtained by the imaging (imaging succeeded) or imaging needs to be performed again (imaging failed). The imaging operator operates the operation section 23 to input the determination result.

If the determination result indicating that the imaging succeeded is input by a predetermined operation of the operation section 23 (step S8: YES), each of a series of frame images obtained in the dynamic imaging is accompanied with information such as an identification ID for identifying the dynamic image, patient information, examination target site, irradiation condition, image reading condition, number indicating the number of imaging and cycle information (for example, the information is written into a header region of the image data), and transmitted to the diagnostic console 3 via the communication section 25 (step S9). Then, the processing ends. On the other hand, if the determination result indicating that the imaging failed is input by a predetermined operation of the operation section 23 (step S8: NO), the series of frame images stored in the storage section 22 is deleted (step S10).

Then, whether to perform re-imaging is determined (step S11). If the imaging operator determines that the re-imaging is necessary (step S11: YES), the processing after step S2 is performed. If the imaging operator determines that the re-imaging is not necessary (step S11: NO), the processing ends.

### [Operation of Diagnostic console 3]

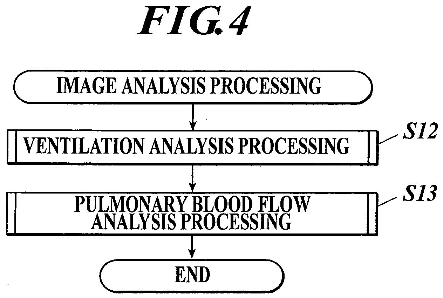

The operation of the diagnostic console 3 will be described. When a series of frame images forming the dynamic image is received from the imaging console 2 via the communication section 35 in the diagnostic console 3, image analysis processing shown in FIG. 4 is executed in cooperation between the control section 31 and the image analysis program stored in the storage section 32.

The diagnostic console 3 does not necessarily perform both ventilation analysis and pulmonary blood flow analysis. The diagnostic console 3 may perform only one of the analyses or other analysis such as the above-mentioned bending and stretching analysis of joints of human body. The details of ventilation analysis and pulmonary blood flow analysis in the image analysis processing are described in Japanese Patent Application Laid Open Publication No.2010-268979, for example.

Hereinafter, a case of performing ventilation analysis (step S12) in the image analysis processing will be briefly described.

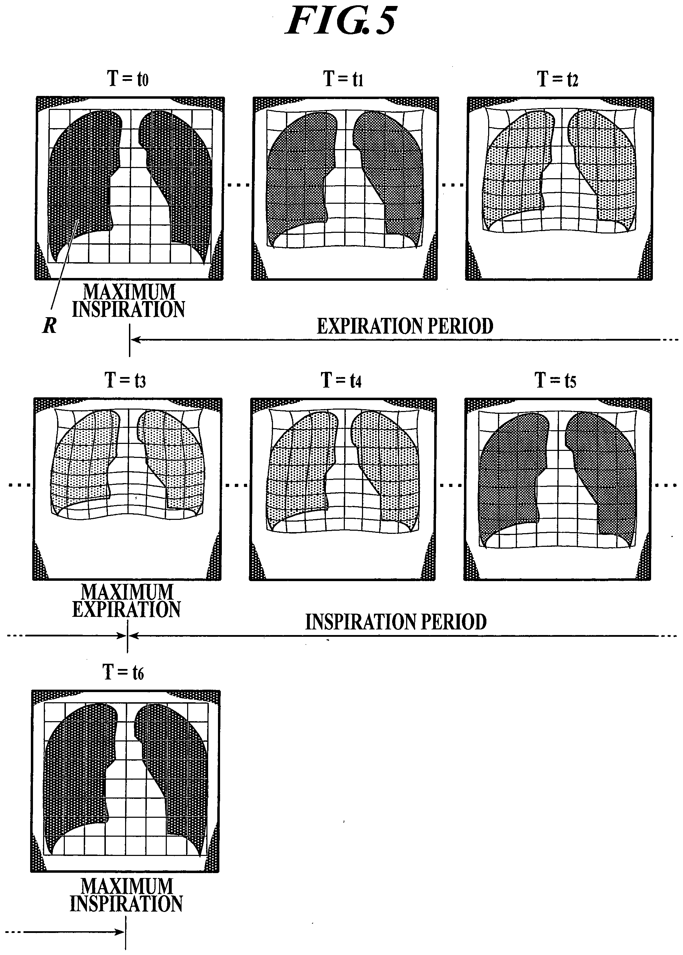

In the ventilation analysis (step S12), the height in vertical direction of the diaphragm is calculated as an index value from the chest dynamic image of the lung field. The diaphragm moves vertically to facilitate lung's breathing motion. For example, as shown in the frame images of a plurality of time phases T (T=t0 to t6) captured in one respiratory cycle which are shown in FIG. 5, the respiratory cycle is formed of an expiration period (T=t0 to t3) when the diaphragm is located at upper positions, and an inspiration period (T=t3 to t6) when the diaphragm is located at lower positions. In such way, in the chest dynamic image, the vertical movement of the diaphragm is an index indicating the breathing motion of lung, and the vertical height of diaphragm is a value of the index (hereinafter, referred to as an index value) indicating the breathing motion of lung.

As shown in FIG. 5, in the frame images, the lung apexes hardly change the vertical position in either the expiration period or the inspiration period. Thus, the height in vertical direction of the diaphragm as the index value can be represented as a vertical distance D between the lung apex and the diaphragm. As shown in FIG. 6, by plotting the distance D along the time t of horizontal axis, the temporal change of vertical height D of the diaphragm can be obtained.

On the basis of the vertical height D of the diaphragm as the index value indicating the breathing motion, diagnosis can be performed as to whether there is abnormality in the ventilation function inside the lung field region R (see FIG. 5), for example. In pulmonary blood flow analysis (step S13) for which the explanation is omitted, it is possible to obtain the temporal change in position of heart wall as an index value indicating the heartbeat, and diagnose whether there is abnormality in pulmonary blood flow on the basis of the change in position (see Japanese Patent Application Laid Open Publication No. 2010-268979).

These analysis results are attached to the data of dynamic image, and stored in the PACS 6 (see FIG. 1), for example.

### [Configuration of Imaging console 2 in the Embodiment]

Hereinafter, the configuration of imaging console 2 in the embodiment will be described. The action of imaging console 2 in the embodiment will also be described together.

The imaging console 2 has a control section, an input section and a display section as shown in FIG. 7. The imaging console 2 can also be configured as a laptop or tablet computer. In the example shown in FIG. 2, the control section 21 including the CPU and such like corresponds to a control section, the operation section 23 such as a mouse and a keyboard corresponds to an input section and the display section 24 corresponds to a display section. Thus, the above sections are respectively referred to as the control section 21, input section 23 and display section 24 as shown in FIG. 7.

As described above, when imaging is performed, the imaging operator inputs patient information and such like of the imaging target (subject M) (see step S1 in FIG. 3), and the imaging console 2 sets the irradiation condition in the irradiation control apparatus 12. The inputting and the setting are performed normally based on the imaging order information.

As shown in FIG. 8, the imaging order information includes patient information such as "patient ID" P2, "patient name" P3, "sex" P4, "age" P5 and "diagnosis department" P6, and imaging site information such as "imaging site" P7 and "imaging direction" P8, for example. The "imaging order ID" P1 is automatically assigned to each set of imaging order information in the order of receiving the imaging order information.

In the example of FIG. 8, the imaging order information of imaging order ID 001 specifies capturing of a moving image (here, dynamic imaging) as seen from the "dynamic state" indicated in the imaging order information of imaging order ID 001. For example, in a case where the imaging is tomosynthesis imaging, the tomosynthesis imaging is specified in the imaging order information. On the other hand, the imaging order information of imaging order IDs 002 and 003 shown in FIG. 8 specifies simple imaging of chest lateral side and abdomen front side, respectively.

When setting an irradiation condition in the irradiation control apparatus 12, the control section 21 of the imaging console 2 sets the irradiation condition such as a pulse rate and an X-ray tube voltage to control the irradiation control apparatus 12 on the basis of the imaging site specified in the imaging order information which was selected by the imaging operator (and also on the basis of patient information in some cases), and controls the irradiation control apparatus 12 to control the irradiation from the radiation source 11.

The imaging order information regarding imaging to be performed is obtained from the RIS 4 (see FIG. 1) or such like prior to the imaging (immediately before the imaging, previous day or such like). The imaging order information may be directly input to the imaging console 2 by the imaging operator. In a case where the imaging console 2 also functions as an RIS client, the input imaging order information is transmitted to the RIS 4 and such like and registered.

This is a general operation which is performed also in simple imaging not only in moving image capturing. However, in the embodiment, the control section 21 of the imaging console 2 determines whether the imaging order information specifies capturing of a moving image (that is, whether the imaging order information specifies "dynamic state" or the like as shown in FIG. 8) when the imaging order information is obtained from the RIS 4 or directly input by the imaging operator.

If the imaging order information specifies capturing of a moving image, the control section 21 of the imaging console 2 obtains a past moving image of the imaging site of the patient from the PACS 6 (see FIG. 1) on the basis of the patient information and the imaging site information specified in the imaging order information. The control section 21 displays the past moving image on the display section 24 according to the operation of the imaging operator.

Such configuration enables the imaging operator to provide an instruction such as "Please breathe deeply" to the patient when the imaging operator watches the past moving image replayed and displayed on the display section 24 of the imaging console 2 and determines that the patient breathes shallowly to make it difficult to examine the lung's ventilation function (see FIG. 5), for example.

Such configuration also enables the imaging operator to perform operation such as setting a value of an X-ray tube voltage to be a higher value by watching the past moving image, the value of X-ray tube voltage being set in the irradiation control apparatus 12 by the imaging console 2.

### [Effect]

As described above, according to the imaging console 2 in the embodiment, when the imaging order information is obtained or input prior to the imaging, the control section 21 obtains the past moving image of the imaging site of the patient specified in the imaging order information, and replays and displays the moving image on the display section 24. Thus, the imaging operator can use the displayed past moving image as a reference, and the present imaging can be performed more appropriately by referring to the past moving image and accurately feeding back the unsuccessful point in the past imaging.

As described in Japanese Patent Application Laid Open Publication No. 2011-152154, in a case where pre-imaging is performed before actual imaging in order to perform the actual imaging appropriately, the exposure dose of the patient is possibly increased for the amount of pre-imaging. However, in the embodiment, the pre-imaging is not performed and the past moving image which has been already captured is used as a reference. Thus, it is possible to accurately avoid a problem of increased exposure dose of patient.

Further, in the embodiment, the control section 21 of the imaging console 2 obtains a past moving image on the basis of patient information and imaging site information specified in the imaging order information and displays the obtained past moving image on the display section 24 at the time when the imaging order information is input to the imaging console 2 not only when the control section 21 obtains the imaging order information from the RIS 4 (see FIG. 1) and such like.

For example, in a case where dynamic imaging needs to be performed urgently, time is required if the imaging order information input to the imaging console 2 is once transmitted to the RIS 4 to be registered and thereafter the imaging order information is obtained from the RIS 4. Thus, it takes time to obtain the past moving image and display the obtained image on the display section 24. However, if the past moving image is obtained at the time when the imaging order information is input to the imaging console 2 as described above, the past moving image can be displayed rapidly on the display section 24, which enables the operator to perform the dynamic imaging urgently.

Hereinafter, configuration examples to which the above embodiment is applied will be described.

### [Configuration Example 1]

In a case where there is no past moving image of the patient who is the target of moving image capturing, or in a case where there is at least no moving image of the same imaging site though there are moving images of the patient, the console 2 may obtain a moving image of another patient which was captured at the imaging site specified in the imaging order information.

Such configuration enables the imaging operator to perform imaging by accurately recognizing problems which will possibly occur at least when imaging is performed at the imaging site though the image is not the moving image of the patient. Thus, the present imaging can be appropriately performed by accurately feeding back the problems in the past moving image.

In the above case, the imaging console 2 may also obtain a moving image of another imaging site of the patient. Such configuration enables the imaging operator to perform imaging accurately recognizing problems which will possibly occur when capturing a moving image of the patient, and thus, the present imaging can be performed appropriately by accurately feeding back the problems generated when the moving image was captured in the past.

### [Configuration Example 2]

The data amount of moving image is a level of gigabyte, and requires much time when transmitting the moving image data from the PACS 6 to the imaging console 2 in some cases. Especially, when the data transmission between the PACS 6 and the imaging console 2 is performed based on the DICOM (Digital Image and Communications in Medicine), the data transmission generally has a low transmission rate and requires time in many cases.

Thus, when it is known in advance that moving image capturing is to be performed for a patient, the past moving image data of the patient is transmitted in advance from the PACS 6 which performs data transmission based on DICOM to the external computer 7 (see FIG. 1) such as a server which has a higher transmission rate.

Then, the imaging console 2 can obtain the past moving image data from the external computer 7 not from the PACS 6 when the imaging order information is obtained or input. By such configuration, data can be obtained in a shorter time than a case of directly obtaining the data from PACS 6 based on the DICOM.

The past moving image data may be stored in each of a plurality of external computers 7 and such like connected to the network N so that the imaging console 2 obtains the past moving image data from an external computer 7 among the plurality of external computers 7 which can transmit the data most rapidly. By such configuration, the past moving image data can be obtained rapidly.

### [Configuration Example 3]

In order to obtain the past moving image data rapidly in a shorter time, the control section 21 of the imaging console 2 may obtain thinned data of past moving image by thinning the past moving image at a predetermined rate, instead of obtaining the whole data of the past moving image.

As the method for thinning an image, there are a temporal thinning method, a spatial thinning method and a temporal and spatial thinning method. The temporal thinning method is a method of extracting an image from among the images forming a moving image at a rate of one for a predetermined number of images (for example, five images), and removing the images which were not extracted (that is, data of the other four images among every five images). The spatial thinning method is a method of extracting data of one pixel from every two or four pixels in each of a plurality of images forming a moving image, and removing the data which was not extracted (that is, data of the other one or three pixel (s) among the every two or four pixels), for example.

The temporal and spatial thinning method is a method of thinning the data by performing both of the temporal thinning method and the spatial thinning method. The rate for thinning data is determined, as in the above methods, so as to be a rate which enables the imaging operator to confirm the unsuccessful point in the past imaging by referring to the thinned past moving image which is displayed on the display section 24 of the imaging console 2.

By such configuration, the imaging operator can perform the present imaging more appropriately by accurately feeding back the unsuccessful point in the past imaging by referring to the thinned past moving image which was obtained by the control section 21 of the imaging console 2 on the basis of the imaging order information and is replayed and displayed on the display section 24. Furthermore, since the data amount obtained by the imaging console 2 is reduced, the past moving image (thinned past moving image in this case) can be obtained rapidly in a shorter time.

### [Configuration Example 4]

When information regarding past moving image data is to be obtained rapidly in a shorter time, instead of obtaining the past moving image data, the control section 21 of the imaging console 2 may obtain the analysis result or information attached to the past moving image (that is, associated with the past moving image) and display the analysis result or information on the display section 24.

As the analysis result attached to the past moving image, in an example of dynamic state analysis of the lung field region R (chest dynamic state analysis), the data of vertical height D of diaphragm shown in FIG. 6 and data of temporal change in position of the heart wall are used, for example. However, there can also be used various analysis results attached to the past moving image such as temporal change in horizontal width of the lung field region R (see FIG. 5), and required time of a defined number of breaths (for example, three breaths), cycle and amplitude of heartbeat, a difference between a maximum value and a minimum value of the vertical height D of diaphragm or horizontal width of the lung field region R which are obtained by further analyzing the above data.

By such configuration, since data amount of the transmitted analysis result is much smaller than the data amount of past moving image, the analysis result as reference information regarding the present imaging can be obtained rapidly in a shorter time than a case of obtaining the past moving image. Since the analysis result is displayed on the display section 24, the imaging operator watches the analysis result of past moving image, and can provide an instruction for appropriate imaging to the patient at the time of imaging, and appropriately change and set the irradiation condition which is to be set in the irradiation control apparatus 12 by the imaging console 2.

The past moving image is accompanied with information noted by a doctor when obtaining the above analysis result by the diagnostic console 3 (see FIG. 1) (for example, the patient breathes shallowly, and heartbeat is weak) and information regarding the cause of failure to obtain the analysis result and findings (for example, the patient breathes very shallowly and the cycle is short, the posture cannot be maintained during the defined number of breaths) in some cases. Also in such cases, the control section 21 of the imaging console 2 may obtain the information attached to the past moving image instead of the past moving image data, and display the information on the display section 24.

In such configuration, since the data amount of transmitted information is much smaller than the data amount of past moving image, the information can be obtained rapidly in a shorter time than a case of obtaining the past moving image. Since the information is displayed on the display section 24, the imaging operator watches the information attached to the past moving image, and can provide an instruction for appropriate imaging to the patient at the time of imaging, perform imaging in the decubitus position for a patient who cannot be in the upright position at the imaging, and appropriately change and set the irradiation condition which is to be set in the irradiation control apparatus 12 by the imaging console 2.

As for a patient having a fast heartbeat which is seen in children or the like, it is also possible to raise the frame rate of imaging , and set an X-ray irradiation time for obtaining images for the defined number of breaths or heartbeats to be shorter.

### [Configuration Example 5]

The above embodiment and configuration examples have been described by taking, as an example, a case where the past moving image is referred to so as to perform imaging appropriately by displaying the past moving image, thinned past moving image, analysis result and information on the display section 24 of the imaging console 2 for the imaging operator to confirm them. However, the control section 21 of the imaging console 2 may automatically determine the irradiation condition to be set in the irradiation control apparatus 12 of the radiation source 11 on the basis of the analysis result or information attached to the past moving image and set the condition in the irradiation control apparatus 12 of the radiation source 11.

Specifically, in a case where chest dynamic imaging is performed for diagnosing lung's ventilation function, moving image capturing is performed for the amount of time when the patient takes a breath three times (defined number of breaths), for example. Thus, when the chest dynamic imaging is specified in the imaging order information, the imaging console 2 sets the irradiation period in the irradiation control apparatus 12 so that the radiation source 11 (see FIG. 2) emits radiation for a period of 15 seconds, for example.

However, in a case where the past moving image is accompanied with an analysis result or information such as "the patient breathes at a short cycle, and the time required for the defined number of breaths (three times) is short (for example 12 seconds)", the control section 21 of the imaging console 2 sets a short irradiation period (for example, 12 seconds) in the irradiation control apparatus 12 of the radiation source 11.

In order to accurately capture images of the temporal change of the lung field region R even if the irradiation period is shorter, the control section 21 of the imaging console 2 sets the pulse rate of radiation emitted from the radiation source 11 to be faster than the pulse rate of a case where the irradiation period is 15 seconds. In this case, as for a frame rate in the image reading condition input to the reading control apparatus 14 (see FIG. 2) by the imaging console 2, for example, a value changed according to the change of pulse rate is input.

Also in a case where the past moving image is accompanied with an analysis result or information such as "the heartbeat cycle is shorter than the heartbeat cycle of a normal patient", the control section 21 of the imaging console 2 can perform the processing similarly.

As described above, since the control section 21 of the imaging console 2 determines the irradiation condition to be set in the irradiation control apparatus 12 of the radiation source 11 on the basis of the analysis result or information attached to the past moving image and sets the irradiation condition in the irradiation control apparatus 12 of the radiation source 11, imaging can be performed appropriately by referring to the past moving image. The determined irradiation condition is also set in the radiation detection section 13 and the reading control apparatus 14 which generate an image by receiving the radiation not only in the irradiation control apparatus 12.

In this case, the determined irradiation condition such as irradiation period and pulse rate (frame rate) of radiation may be displayed on the display section 24 of the imaging console 2 so that the irradiation condition can be changed and set by the imaging operator approving the condition.

It goes without saying that the present invention is not limited to the above embodiment and configuration examples, and can be changed appropriately within the scope of the present invention.

## Claims

1. An imaging console (2) comprising:
a display section (24) which is capable of displaying a moving image; and
a control section (21) which performs control on the basis of imaging order information regarding imaging to be performed, the imaging order information specifying at least patient information (P2 to P6) regarding a patient and imaging site information (P7 and P8) regarding an imaging site; wherein
in a case where the imaging order information specifies imaging of a moving image, at the time when the imaging order information is obtained or input, the control section (21) obtains a past moving image of the patient or a past moving image of the imaging site on the basis of at least one of the patient information (P2 to P6) and the imaging site information (P7 and P8) specified in the imaging order information, and displays the obtained past moving image on the display section (24).

2. The imaging console (2) according to claim 1, wherein the control section (21) obtains thinned data of the past moving image instead of data of the past moving image, and displays the thinned data of the past moving image on the display section (24), the thinned data being obtained by thinning the past moving image at a predetermined rate.

3. The imaging console (2) according to claim 1, wherein the control section (21) obtains an analysis result(D) or information attached to the past moving image instead of the past moving image, and displays the analysis result (D) or the information of the past moving image on the display section.

4. A radiation imaging system (100), comprising:
the imaging console (2) according to claim 3;
a radiation source (11) which emits radiation to a semiconductor image sensor (13) through a subject (M);
a radiation detection section (13) which detects radiation transmitted through the subject (M) according to an intensity thereof, converts the detected radiation into an electrical signal for each pixel, and accumulates the converted electrical signal; and
a reading control apparatus (14) which reads out the electrical signal accumulated in each pixel of the radiation detection section (13) and obtains image data, wherein
the control section (21) of the imaging console (2) determines an irradiation condition to be set in the radiation source (11) on the basis of the analysis result (D) or the information attached to the past moving image, and sets the irradiation condition in an irradiation control apparatus (12) which controls the radiation source (11), the radiation detection section (13) and the reading control apparatus (14).
